(19) Europäisches Patentamt
European Patent Office
Office européen des brevets

(11) Veröffentlichungsnummer : **0 054 158 B1**

(12) **EUROPÄISCHE PATENTSCHRIFT**

(45) Veröffentlichungstag der Patentschrift :
26.10.83

(51) Int. Cl.³ : **B 01 J 23/44, B 01 J 21/18, C 07 C 67/10**

(21) Anmeldenummer : 81109062.0

(22) Anmeldetag : 28.10.81

(54) Palladium(II)-Katalysator, seine Herstellung und Verwendung.

(30) Priorität : 16.12.80 DE 3047347

(43) Veröffentlichungstag der Anmeldung :
23.06.82 Patentblatt 82/25

(45) Bekanntmachung des Hinweises auf die Patenterteilung : 26.10.83 Patentblatt 83/43

(84) Benannte Vertragsstaaten :
DE FR GB IT NL

(56) Entgegenhaltungen :
DE A 1 943 297
DE B 1 127 888
GB A 1 187 231
US A 2 586 812
US A 3 804 779

CHEMICAL ABSTRACTS, Band 69, 1968, Seite 5464, Zusammenfassung 58799h, COLUMBUS, OHIO (US) K.A. GALUTKINA et al. : « Vinyl exchange reaction on a palladium catalyst »

(73) Patentinhaber : WACKER-CHEMIE GMBH
Prinzregentenstrasse 22
D-8000 München 22 (DE)

(72) Erfinder : Blum, Klaus, Dr.
Hermann-Hiller-strasse 47
D-8263 Burghausen (DE)
Erfinder : Strasser, Rudolf, Dr. Dipl.-Chem.
Lindacher Strasse 58
D-8263 Burghausen (DE)

EP 0 054 158 B1

Palladium(II)-Katalysator, seine Herstellung und Verwendung

Palladium(II)-Salz-Katalysatoren mit Aktivkohle als Trägermaterial sind an sich bekannt. Beispielsweise wird gemäß CA *69*, 1968, 58799 h ein derartiger Katalysator zur Vinylierung von Propionsäure mit Vinylacetat vorgestellt. Es wird eine Standzeit von zumindest 250 Stunden angegeben. Die Aktivität dieses Katalysators ist jedoch, berücksichtigt man die angegebene Verweildauer des Reaktionsgemisches von 3 bis 3,5 Stunden, für eine industrielle Nutzung zu gering.

Ferner werden gemäß DE-PS 11 27 888 Palladium(II)-Salz-Katalysatoren beschrieben zur Herstellung von Vinylestern höherer Carbonsäuren, für die ebenfalls Aktivkohle als Trägermaterial empfohlen wird. Nachteilig an derartigen Katalysatoren ist ihre geringe Standzeit : nach einer Betriebsdauer von ca. 100 Stunden müssen schon Umsatzeinbußen von ca. 10 % hingenommen werden. Darüberhinaus treten selbst nach sehr kürzer Betriebsdauer (ca. 20 Stunden) Palladiumsalzverluste auf.

Aufgabe der Erfindung war es, Palladium(II)-Salz-Katalysatoren mit erhöhter Lebensdauer zu entwickeln.

Es wurde nun gefunden, daß diese Aufgabe gelöst wird, indem als Trägermaterial für den Palladium(II)-Salz-Katalysator Aktivkohle mit einem analytischen $SiO_2$-Gehalt von zumindest 0,5 Gew.% verwendet wird.

Gegenstand der Erfindung sind Trägerkatalysatoren auf Basis von Palladium(II)-Salz und Aktivkohle, die dadurch gekennzeichnet sind, daß das Trägermaterial Aktivkohle mit einem analytischen $SiO_2$-Gehalt von 0,5 bis 8 Gew.% ist.

Vorzugsweise liegt der $SiO_2$-Gehalt bei 0,8 bis 5,0 Gew.%.

Der Palladium-Gehalt der erfindungsgemäßen Trägerkatalysatoren bewegt sich in den an sich bekannten Grenzen. Ausgezeichnete Ergebnisse werden erzielt bei einem Gehalt von 2 bis 4 Gew.%, bezogen auf Palladium.

Günstigerweise werden als Palladium(II)-Salze für die erfindungsgemäßen Katalysatoren zumindest teilweise Doppelsalze des Palladium(II) eingesetzt, wobei als Zusatzkomponenten die Salze von Alkalimetallen, insbesondere des Lithiums, Natriums, Kaliums, von Erdalkalimetallen, beispielsweise von Magnesium, Kalzium, Barium, sowie von Seltenerdmetallen, beispielsweise von Neodym, Cer, Lanthan, Dysprosium in Betracht kommen.

Aktivkohlen mit einem analytischen $SiO_2$-Gehalt von 0,5 bis 8 Gew.% sind im Handel erhältlich. Ihre Herstellung kann beispielsweise, ausgehend von $SiO_2$-freien bzw. $SiO_2$-armen Aktivkohlen, durch Imprägnieren des Materials mit Wasserglas und dergleichen und anschließendes Ausheizen des imprägnierten Materials, erfolgen. Erfindungsgemäß einzusetzende Aktivkohlen können jedoch auch z. B. aus Steinkohlen, die einen entsprechenden $SiO_2$-Gehalt aufweisen, erhalten werden, nachdem sie in an sich bekannter Weise durch Temperaturbehandlung in

Aktivkohlen übergeführt wurden.

Der analytische Gehalt von $SiO_2$ der Aktivkohlen wird nach Veraschen des Materials in üblicher Weise bestimmt.

Die Herstellung der erfindungsgemäßen Katalysatoren erfolgt durch Imprägnieren des entsprechenden Trägermaterials mit Palladium(II)-Salzlösung.

Ein bevorzugtes Herstellungsverfahren ist dadurch gekennzeichnet, daß Aktivkohle mit einem analytischen $SiO_2$-Gehalt von 0,5 bis 8 Gew.% mit Palladium(II)-Lösung imprägniert wird, die einen pH-Wert von 1 bis 6, vorzugsweise 2 bis 5, aufweist.

Unter Imprägnieren wird eine Behandlung von Aktivkohle mit Palladium(II)-Lösung verstanden, die derart erfolgt, daß Palladium(II) an Aktivkohle adsorbiert wird. Dies erfolgt beispielsweise durch Schütteln, Rühren, Stehenlassen und dergleichen einer Suspension von Aktivkohle in Palladium(II)-Salzlösung.

Üblicherweise wird von stark salzsaurer Pd(II)Cl$_2$-Lösung ausgegangen, die durch Basenzusatz auf einen pH-Wert von 1 bis 6 gebracht wird. Als Basen kommen in erster Linie die Oxide und Hydroxide der Alkali-, Erdalkali- und Seltenerdmetalle in Betracht. Auf diese Weise gelingt es in einfacher Weise, die Zusätzkomponente zum Palladium-Doppelsalz einzuführen. Grundsätzlich können jedoch derartige Zusatzkomponenten auch in Form von beispielsweise Neutralsalzen eingeführt werden.

Die Adsorption der Salzkomponenten an die Aktivkohle kann weiterhin durch Zugabe organischer Verbindungen, wie Aceton und dergleichen, zur Pd(II)-Salz-Lösung gefördert werden.

Die erfindungsgemäßen Katalysatoren zeichnen sich gegenüber herkömmlichen Palladium(II)-Salz/Aktivkohle-Katalysatoren durch erhöhte Standzeiten aus. Die hinlänglich bekannten Palladium(II)-Salz-Verluste sind selbst bei längeren Betriebszeiten minimal.

Die erfindungsgemäßen Katalysatoren fördern die Alkenylierung von Carbonsäuren mit niederen Carbonsäurealkenylestern.

Gegenstand der Erfindung ist somit weiterhin ein Verfahren zur Herstellung von Carbonsäurealkenylestern durch Umsetzen von Carbonsäuren mit niederen Carbonsäurealkenylestern, das dadurch gekennzeichnet ist, daß Trägerkatalysatoren auf Basis von Palladium(II)-Salz und Aktivkohle eingesetzt werden, wobei die Aktivkohle einen analytischen $SiO_2$-Gehalt von 0,5 bis 8 Gew.% aufweist.

Die Alkenylierungsreaktionen werden in der Regel beim Druck der umgebenden Atmosphäre bei Temperaturen von 40 bis 100 °C durchgeführt.

Da es sich um Gleichgewichtsreaktionen handelt, wird zweckmäßigerweise das Alkenylierungsmittel im Überschuß zugesetzt. Das molare Verhältnis von Carbonsäurealkenylester zu Carbonsäure beträgt 1 : 1 bis 5 : 1, vorzugsweise etwa 3 : 1.

Als Alkenylierungsmittel werden die niederen Carbonsäurealkenylester eingesetzt. Beispiele sind u. a. Vinylacetat, Allylacetat, Methallylacetat, Propenylacetat, Isopropenylacetat, Vinylpropionat, insbesondere Vinylacetat.

Beispiele für Carbonsäuren sind Propionsäure, Buttersäure, Valeriansäure, Caprylsäure, Caprinsäure, Laurinsäure, Palmitinsäure, Stearinsäure, Crotonsäure, Sorbinsäure, Ölsäure, Cyclohexancarbonsäure, Benzoesäure, Pivalinsäure, α-Chlorpropionsäure, Adipinsäure und Capronsäure.

Die Umsetzungen können kontinuierlich oder diskontinuierlich erfolgen. Eine kontinuierliche Arbeitsweise erfolgt beispielsweise in einer beheizbaren Säulenanordnung, analog einer Anordnung für Säulenchromatographie, wobei durch Zulauf- und Ablaufgeschwindigkeit der Reaktionsmischung die Verweildauer der Reaktanten gesteuert werden kann.

Nach dem erfindungsgemäßen Verfahren gelingt es, Alkenylierungsreaktionen von Carbonsäuren in hoher Raum-Zeit-Ausbeute auszuführen.

Die Erfindung wird nun anhand von Beispielen näher erläutert :

Beispiel 1

Herstellung des Katalysators :
1,16 g Palladiumchlorid werden in 2 ml konzentrierter Salzsäure aufgelöst und mit 30 ml destilliertem Wasser verdünnt. Durch Zugabe von 11,4 ml 1 nNaOH wird auf einen pH-Wert von 3 eingestellt.

Anschließend wird mit 30 ml Aceton versetzt. In der so erhaltenen Lösung werden nun 20 g gekörnter Aktivkohle mit einem analytischen $SiO_2$-Gehalt von 0,9 Gew.% aufgeschlämmt. Nach ca. 10 Minuten ist die Lösung entfärbt. Die auf diese Weise mit Palladium(II)-Salz imprägnierte Aktivkohle wird nun abfiltriert und schließlich bei 80 °C im Stickstoffstrom getrocknet.

Beispiel 2

1,16 g $PdCl_2$ und 1,8 g $NdCl_3$ werden in 3,5 ml konzentrierter Salzsäure aufgelöst und mit 11,5 ml 1 nNaOH versetzt. Der pH-Wert der Lösung beträgt 2,0. Schließlich werden noch 30 ml Aceton beigegeben.

20 g einer gekörnten Aktivkohle mit einem analytischen $SiO_2$-Gehalt von 0,9 Gew.% werden nun mit der so bereiteten Lösung in der gleichen Weise behandelt, wie gemäß Beispiel 1 beschrieben.

Beispiel 3

Die Arbeitsweise gemäß Beispiel 1 wird wiederholt mit der Abänderung, daß eine gekörnte Aktivkohle mit einem analytischen $SiO_2$-Gehalt von 2,0 Gew.% eingesetzt werden.

Beispiel 4

Die Arbeitsweise gemäß Beispiel 3 wird wiederholt mit der Abänderung, daß anstatt mit NaOH auf einen pH-Wert von 3 mit KOH auf einen pH-Wert von 5 gestellt wird.

Beispiel 5

Die Arbeitsweise gemäß Beispiel 1 wird wiederholt mit der Abänderung, daß eine gekörnte Aktivkohle mit einem analytischen $SiO_2$-Gehalt von 4,9 Gew.% eingesetzt wird.

Vergleichsbeispiel 1

Die Arbeitsweise gemäß Beispiel 1 wird wiederholt, mit der Abänderung, daß eine gekörnte Aktivkohle mit einem analytischen $SiO_2$-Gehalt von 0,15 Gew.% eingesetzt wird.

Vergleichsbeispiel 2

Die Arbeitsweise gemäß Beispiel 3 wird wiederholt, mit der Abänderung, daß anstelle von NaOH 0,66 g NaCl eingesetzt wird und der pH-Wert unter 0 liegt.

Beispiel 6

20 g des gemäß Beispiel 1 hergestellten Katalysators werden in eine beheizbare Säule gefüllt. Die Temperatur innerhalb der Säule wird auf 62 °C gehalten. Es wird ein vorgewärmtes Gemisch aus Vinylacetat und Laurinsäure im Molverhältnis 3 : 1 aus einem auf die Säule aufgesetzten Tropftrichter kontinuierlich beigegeben und das die Säule verlassende Reaktionsgemisch nach Art einer Säulenchromatographie so abgezogen, daß eine Verweildauer des Reaktionsgemisches innerhalb der Katalysatorzone von 46 Minuten entsteht.

Der Umsatz zu Vinyllaurat betrug anfänglich, bezogen auf eingesetzte Laurinsäure, 71 %, nach einer Standzeit von 700 Stunden 55 % und nach 1 080 Stunden immer noch 41 %. Entsprechend ergibt sich nach einer Standzeit von 1 080 Stunden des Katalysators ein mittlerer Umsatz von 57 %.

Beispiel 7

Die Arbeitsweise gemäß Beispiel 6 wird wiederholt, mit der Abänderung, daß ein Katalysator gemäß Beispiel 5 eingesetzt wird.

Der Umsatz zu Vinyllaurat, bezogen auf umgesetzte Laurinsäuremenge, lag anfänglich bei 72 %, nach 600 Stunden bei 60 % und nach 1 160 Stunden noch bei 41 %. Entsprechend ergibt sich ein mittlerer Umsatz von 59 % bei 1 160 Stunden Betriebsdauer.

Beispiel 8

Die Arbeitsweise gemäß Beispiel 6 wird wiederholt, mit der Abänderung, daß ein Katalysator gemäß Beispiel 3 eingesetzt wird.

Die Verweilzeit des Reaktionsgemisches innerhalb der Katalysatorzone betrug 42 Minuten. Der Umsatz lag nach 600 Stunden noch bei 70 %, nach 1 240 Stunden noch bei 40 %. Entsprechend ergibt sich ein mittlerer Umsatz bei einer Standzeit von 1 240 Stunden von 63,5 %.

Beispiel 9

Es wird die Arbeitsweise gemäß Beispiel 6 wiederholt, mit der Abänderung, daß ein Katalysator gemäß Beispiel 2 eingesetzt wird.

Der mittlere Umsatz nach 858 Stunden betrug 58 %.

Vergleichsbeispiel 3

Es wird die Arbeitsweise gemäß Beispiel 6 wiederholt, mit der Abänderung, daß ein Katalysator gemäß Vergleichsbeispiel 1 eingesetzt wird.

Der mittlere Umsatz zu Vinyllaurat, bezogen auf eingesetzte Laurinsäure, betrug nach 67 Stunden 35 %.

Vergleichsbeispiel 4

Es wird die Arbeitsweise gemäß Beispiel 6 wiederholt, mit der Abänderung, daß ein Katalysator gemäß Vergleichsbeispiel 2 eingesetzt wird.

Der Umsatz zu Vinyllaurat fiel von anfänglich 75 % nach 263 Stunden auf 60 % und nach 890 Stunden auf 41 %.

Die Gesamtleistung des Katalysators erreicht somit nur ca. 77 % der Leistung des Katalysators gemäß Beispiel 3.

Beispiel 10

Es wird der Katalysator gemäß Beispiel 4 eingesetzt.

Ein Gemisch aus 2 Teilen Vinylacetat und 1 Teil Capronsäure wird bei einer Verweilzeit von 43 Minuten analog Beispiel 6 über den Kontakt geleitet.

Der Umsatz zu Vinylcapronat blieb über 450 Stunden konstant bei 45 %. Nach dieser Standdauer besaß der Katalysator noch seine volle Aktivität.

Beispiel 11

Es wird ein Katalysator analog Beispiel 3 eingesetzt mit 2,0 Gew.% SiO$_2$-Gehalt, wobei jedoch beim Herstellungsprozeß nicht mit 1 nNaOH, sondern mit 1 nKOH auf einen pH-Wert von 3 gestellt wurde.

Vinylacetat und Capronsäure im molaren Verhältnis 2 : 1 wurden bei einer Verweildauer von 42 Minuten in der gemäß Beispiel 6 beschriebenen Anordnung bei 62 °C umgesetzt.

Der Umsatz von Capronsäure zu Capronsäurevinylester lag bei 53 %.

Beispiel 12

In Gegenwart von 20 g Katalysator gemäß Beispiel 3 wurden 3 Mol Vinylacetat und 1 Mol Benzoesäure bei 100 °C unter Rückfluß eine Stunde lang gekocht.

Der Umsatz von Benzoesäure zu Vinylbenzoat betrug 58 %.

Der Katalysator war nach Abtrennen vom Reaktionsgemisch ohne Beeinträchtigung wieder einsetzbar.

Ansprüche

1. Trägerkatalysator auf Basis von Palladium(II)-Salz und Aktivkohle, dadurch gekennzeichnet, daß das Trägermaterial Aktivkohle mit einem analytischen SiO$_2$-Gehalt von 0,5 bis 8 Gew.% ist.

2. Katalysator nach Anspruch 1, dadurch gekennzeichnet, daß der SiO$_2$-Gehalt 0,8 bis 5,0 Gew.% beträgt.

3. Katalysator nach den Ansprüche 1 und 2, dadurch gekennzeichnet, daß der Palladium(II)-Gehalt, bezogen auf das Gesamtgewicht des Katalysators 2 bis 4 Gew.% beträgt.

4. Verfahren zur Herstellung des Katalysators nach den Ansprüchen 1, 2 und 3, dadurch gekennzeichnet, daß Aktivkohle mit einem analytischen SiO$_2$-Gehalt von 0,5 bis 8 Gew.% mit Palladium(II)-Lösung imprägniert wird, die einen pH-Wert von 1 bis 6 aufweist.

5. Verfahren zur Herstellung von Carbonsäurealkenylestern durch Umsetzen von Carbonsäure mit niederen Carbonsäurealkenylestern, dadurch gekennzeichnet, daß Katalysatoren nach den Ansprüchen 1 bis 3 eingesetzt werden.

Claims

1. Supported catalyst based on a palladium (II) salt and active carbon, characterised in that the support material is active carbon having an analytical SiO$_2$ content of from 0.5 to 8 % by weight.

2. Catalyst according to claim 1, characterised in that the SiO$_2$ content is from 0.8 to 5 % by weight.

3. Catalyst according to claims 1 and 2, characterised in that the palladium (II) content is from 2 to 4 % by weight, based on the total weight of the catalyst.

4. Process for the manufacture of the catalyst according to claims 1, 2 and 3, characterised in that active carbon having an analytical SiO$_2$ content of from 0.5 to 8 % by weight is impregnated with a palladium (II) solution having a pH of from 1 to 6.

5. Process for the manufacture of alkenyl esters of carboxylic acids by reacting carboxylic acids with alkenyl esters of lower carboxylic acids, characterised in that catalysts according to claims 1 to 3 are used.

**Revendications**

1. Catalyseur sur support à base d'un sel de palladium (II) et de charbon actif, caractérisé en ce que le charbon actif servant de support a une teneur analytique en $SiO_2$ de 0,5 à 8 % en poids.

2. Catalyseur selon la revendication 1 caractérisé en ce que la teneur en $SiO_2$ du charbon actif est de 0,8 à 5,0 % en poids.

3. Catalyseur selon la revendication 1 ou 2, caractérisé en ce que sa teneur en palladium divalent, rapportée au poids total du catalyseur, est de 2 à 4 % en poids.

4. Procédé de préparation d'un catalyseur selon l'une quelconque des revendications 1 à 3, caractérisé en ce que l'on imprègne un charbon actif ayant une teneur analytique en $SiO_2$ de 0,5 à 8 % en poids avec une solution d'un sel palladeux à un pH de 1 à 6.

5. Procédé de préparation d'esters alcényliques d'acides carboxyliques par réaction de l'acide avec des esters alcényliques d'acides carboxyliques inférieurs, procédé caractérisé en ce que l'on utilise un catalyseur selon l'une quelconque des revendications 1 à 3.